# EUROPÄISCHE PATENTSCHRIFT

(11) **EP 2 342 592 B1**
(45) Veröffentlichungstag und Bekanntmachung des Hinweises auf die Patenterteilung: **27.02.2013**
(21) Anmeldenummer: 09771673.2
(22) Anmeldetag: 16.10.2009
(51) Int. Cl.: G02B 6/00, A61B 19/00

(54) **LICHTQUELLE**
LIGHT SOURCE
SOURCE DE LUMIÈRE

(30) Priorität: 10.11.2008 DE 102008056565
(43) Veröffentlichungstag der Anmeldung: 13.07.2011
(73) Patentinhaber: Geuder AG, 69126 Heidelberg (DE)
(72) Erfinder: GEUDER, Volker, 69126 Heidelberg (DE); DRAHEIM, René, 69207 Sandhausen (DE)
(74) Vertreter: Ullrich & Naumann
(86) Internationale Anmeldenummer: PCT/DE2009/001443
(87) Internationale Veröffentlichungsnummer: WO 2010/051787

(56) Entgegenhaltungen:
- DE-A1- 19 615 678
- DE-A1-102006 051 736
- US-A1- 2005 282 102

## Beschreibung

Die Erfindung betrifft eine Lichtquelle zum Einkoppeln von Licht in ein medizinisches Gerät, insbesondere in ein chirurgisches Handgerät, vorzugsweise zur Anwendung in der Ophthalmologie, mit einem Leuchtmittel, einem sich vom Leuchtmittel erstreckenden Lichtleiter und einer Stromversorgung für das Leuchtmittel.

Lichtquellen zum Einkoppeln von Licht in ein medizinisches Gerät, insbesondere auch in ein chirurgisches Handgerät sind aus der Praxis hinlänglich bekannt. So werden solche Lichtquellen beispielsweise in der Ophthalmologie verwendet, wobei regelmäßig eine stationäre Lichtquelle vorgesehen ist, von der aus das Licht regelmäßig über eine Lichtleitfaser in ein Handgerät eingekoppelt wird, um beispielsweise einen Operationsbereich am/im Auge zu beleuchten. Eine solche Lichtquelle umfasst ein Leuchtmittel, wobei es sich dabei um Leuchtmittel beliebiger Art handeln kann. Wie bereits zuvor erwähnt, ist ein Lichtleiter vorgesehen, der sich vom Leuchtmittel aus dorthin erstreckt, wo das Licht benötigt wird. Des Weiteren ist im Stand der Technik eine meist integrierte Stromversorgung für das Leuchtmittel vorgesehen, wobei dort die Lichtquelle nebst Stromversorgung eine bauliche Einheit bildet.

Lichtquellen der gattungsbildenden Art bieten wenig Flexibilität, da es sich dabei regelmäßig um Standgeräte handelt, die üblicherweise an ein stationäres Stromnetz anzuschließen sind. Außerdem beanspruchen die Lichtquellen der gattungsbildenden Art einen erheblichen Raum und sind auch insoweit wenig flexibel. Benötigt man Beleuchtungslicht unterschiedlicher Wellenlängen, sind aufwendige Filter oder ist ein Austausch der Lichtquelle erforderlich. Dies bedingt einen konstruktiven Aufwand und ist in der Handhabung umständlich.

Der vorliegenden Erfindung liegt daher die Aufgabe zugrunde, eine Lichtquelle zum Einkoppeln von Licht in ein medizinisches Gerät, insbesondere in ein chirurgisches Handgerät, vorzugsweise zur Anwendung in der Ophthalmologie, derart auszugestalten und weiterzubilden, dass es sich bei kleinster Bauweise flexibel einsetzen lässt. Die Handhabung der Lichtquelle soll dabei äußerst einfach sein.

Die voranstehende Aufgabe ist erfindungsgemäß durch eine Lichtquelle mit den Merkmalen des Patentanspruchs 1 gelöst. Danach ist die gattungsbildende Lichtquelle dadurch gekennzeichnet, dass das Leuchtmittel in einem adapterartigen Gehäuse angeordnet ist, das einen Anschluss für die Stromversorgung und einen Anschluss für den Lichtleiter umfasst. Patent DE 19615678 A1 offenbart den Gegenstand des Oberbegriffs des Anspruchs 1.

Erfindungsgemäß ist erkannt worden, dass es sinnvoll ist, von der bisher üblichen Ausgestaltung eines Leuchtmittels nebst Stromversorgung in einem gemeinsamen Gehäuse abzuweichen. Vielmehr liegt der Erfindung die Idee zugrunde, das Leuchtmittel in einem adapterartigen Gehäuse anzuordnen, welches unabhängig von der Stromversorgung als bauliche Einheit zu handhaben ist. Dazu ist das Leuchtmittel in einem adapterartigen Gehäuse angeordnet und umfasst zwei Anschlüsse, nämlich einen Anschluss für die Stromversorgung und einen Anschluss für den Lichtleiter.

Die erfindungsgemäße Lichtquelle ist mit dem im adapterartigen Gehäuse angeordneten Leuchtmittel derart ausgelegt, dass das Leuchtmittel bzw. der Adapter beliebig austauschbar ist. Ebenso lässt sich an den Adapter eine mobile Stromversorgung anschließen. Gleichermaßen dient der Adapter zum Ankoppeln eines Lichtleiters, der dann wiederum unmittelbar mit einem medizinischen Handgerät oder dgl. verbunden ist.

Grundsätzlich ist es auch denkbar, dass der das Leuchtmittel enthaltende Adapter unmittelbar an das Handgerät angeflanscht wird. Rückseitig des Adapters kann die Stromversorgung in einem separaten Gehäuse angeordnet sein. Ebenso ist es denkbar, dass der Adapter an dem Gehäuse einer mobilen Stromversorgung angekoppelt ist und sich zwischen dem Adapter und dem Handgerät ein Lichtleiter erstreckt. Beide Varianten sind grundsätzlich denkbar und durch den Erfindungsgedanken abgedeckt.

Im Konkreten kann das den Adapter bildende Gehäuse mit dem darin befindlichen Leuchtmittel einteilig ausgeführt sein. Das Gehäuse kann beliebige Formen haben, ist jedoch vorzugsweise in Form einer zylindrischen Hülse ausgeführt.

Dabei ist es von weiterem Vorteil, wenn die Anschlüsse für die Stromversorgung und den Lichtleiter auf gegenüberliegenden Seiten des Gehäuses angeordnet sind. Ein Anschluss für die Stromversorgung ist dabei als dem Gehäuse zugeordneter Stecker ausgebildet, der endseitig aus dem Gehäuse bzw. aus dem Adapter herausragt. Dabei kann es sich um einen handelsüblichen Schwachstromstecker zur Leistungsaufnahme bzw. zum elektrischen Anschluss an die Stromversorgung handeln. Jedenfalls ist es von Vorteil, wenn der Stecker endseitig aus dem Gehäuse zumindest geringfügig herausragt, so dass eine Steckverbindung beispielsweise mit einem Akku-Pack möglich ist. Zur Vermeidung einer unbeabsichtigten Beschädigung könnte eine Schutzkappe für den Stecker vorgesehen sein. Auch ist es denkbar, dass im Bereich des aus dem Gehäuse herausragenden Steckers eine um das Adaptergehäuse herum angeordnete Hülse vorgesehen ist, die beispielsweise gegen die Kraft einer Feder zurückschiebbar ist, wobei dies beim Einstecken des Steckers in eine entsprechende Buchse der Stromversorgung - quasi automatisch - geschehen kann.

Beim Anschluss für den Lichtleiter ist es von Bedeutung, dass dieser endseitig unmittelbar bis an das Leuchtmittel heranführbar ist. Dazu könnte dieser Anschluss als eine in das Gehäuse integrierte Aufnahme für das Koppelende eines Lichtleiters oder eines Lichtleiterbündels ausgeführt sein. Im Inneren des Gehäuses bzw. des Adapters könnte in Form einer inneren Schulter ein Anschlag vorgesehen sein, an den sich das Koppelende des Lichtleiters/Lichtleiterbündels heranschieben lässt. Gleich danach schließt sich die im Gehäuse angeordnete Lichtquelle oder eine dazwischen wirkende Optik an.

Wesentlich ist jedenfalls, dass die im Gehäuse ausgebildet Aufnahme für den Lichtleiter bzw. für das Lichtleiterbündel im Wesentlichen durch die Innenwandung des Gehäuses definiert wird.

Zur Vermeidung eines unbeabsichtigten Herausrutschens des Lichtleiters ist es von Vorteil, wenn Mittel zum Arretieren des Lichtleiters in seiner angekoppelten Position vorgesehen sind. Dabei kann es sich um Rastmittel bzw. Klemmmittel jedweder Art handeln. In konstruktiver Hinsicht ist es ganz besonders einfach, wenn als Klemmmittel eine von außerhalb des Gehäuses betätigbare Schraube, beispielsweise eine Madenschraube, vorgesehen ist, die sich zumindest geringfügig gegen das Koppelende des Lichtleiters schrauben bzw. drücken lässt. Eine Sicherung des Lichtleiters innerhalb des Adapters ist dadurch gewährleistet. Beliebige andere Anschlüsse, beispielsweise auch Bajonettanschlüsse, sind realisierbar.

Das Gehäuse bzw. der Adapter kann entsprechend dem Leuchtmittel unterschiedlich groß ausgeführt sein. Insbesondere im Hinblick auf eine Miniaturisierung des Adapters ist es von ganz besonderem Vorteil, wenn es sich bei dem Leuchtmittel um eine LED oder eine Anordnung aus mehreren LEDs handelt. Auch lassen sich RGB-LEDs mit entsprechender Steuerung der Farbauswahl integrieren. Grundsätzlich ist es denkbar, Adapter mit LEDs unterschiedlicher Wellenlänge im Emissionsbereich vorzusehen.

Wie bereits zuvor erwähnt, können innerhalb des Gehäuses zwischen dem Leuchtmittel und der Ankoppelstelle für den Lichtleiter eine Linse bzw. ein Linsensystem, ein Filter oder sonstige optisch wirkende Mittel angeordnet sein. Auch ist es denkbar, Adapter mit unterschiedlichen optischen Mitteln vorzusehen oder die optischen Mittel durch einen kleinen Einschubschacht austauschbar zu gestalten, und zwar je nach Bedarf. Wesentlich ist es jedenfalls, dass die optischen Mittel dem Leuchtmittel nachgeordnet sind, wobei diese das in den Lichtleiter einzukoppelnde Licht beeinflussen.

Gerade bei kleinstmöglicher Bauweise ist es von Vorteil, wenn dem Leuchtmittel ein Kühlkörper zugeordnet ist. Dieser Kühlkörper kann als unabhängige bauliche Einheit innerhalb des Gehäuses/Adapters angeordnet sein. Ebenso ist es denkbar, dass der Kühlkörper als wärmeleitendes Mittel zwischen dem Leuchtmittel und der Wandung des Gehäuses dient, so dass die Wärme unmittelbar über das Gehäuse nach außen abgeführt wird. Dies bietet sich besonders dann an, wenn es sich bei dem Gehäuse um ein metallisches Gehäuse mit guter Wärmeleitung handelt.

An dieser Stelle sei angemerkt, dass der das Leuchtmittel umfassende Adapter unabhängig handhabbar ist, wobei zum Betreiben des Adapters einerseits eine Stromversorgung und andererseits ein Lichtleiter zum Einkoppeln des Lichts erforderlich ist. Beliebige Anwendungen lassen sich durch eine solche vorteilhafte Konstruktion ableiten.

Bei der Stromversorgung kann es sich um eine ortsfeste Einheit handeln. Insbesondere zur Begünstigung der Mobilität ist es jedoch von Vorteil, wenn die Stromversorgung in einem separaten, möglichst kleinen Gehäuse untergebracht ist, vorzugsweise im Sinne eines Akku-Packs. Entsprechend umfasst das Gehäuse der Stromversorgung eine Buchse zum Einstecken des Steckers des das Leuchtmittel umfassenden Gehäuses bzw. Adapters. Die Buchse des Gehäuses der Stromversorgung und der Stecker des Gehäuses des Leuchtmittels können dabei derart aufeinander abgestimmt sein, dass das Gehäuse des Leuchtmittels bei eingestecktem Stecker am Gehäuse der Stromversorgung vollflächig zur Anlage kommt, wodurch sich eine stabile bauliche Einheit ergibt, die allerdings zum Austausch der Stromversorgung oder des Adapters leicht entkoppelbar ist. So lässt sich jederzeit eine Stromversorgung mit frisch aufgeladenen Akkus anstecken oder ein Adapter mit andersartiger Lichtquelle ankoppeln.

Wie beim Gehäuse des Leuchtmittels kann die Buchse in das Gehäuse der Stromversorgung integriert sein. Auch ist es denkbar, dass das Gehäuse der Stromversorgung eine auf den Querschnitt des Gehäuses des Leuchtmittels abgestimmte Nische umfasst, in die sich das Gehäuse des Leuchtmittels zumindest geringfügig einstecken lässt. Beliebige mechanische und auch magnetische Kopplungsmöglichkeiten zwischen den beiden Gehäusen sind denkbar. Auch ist es grundsätzlich möglich, bei einem Wechselstrombetrieb die Energieankopplung induktiv zu gestalten.

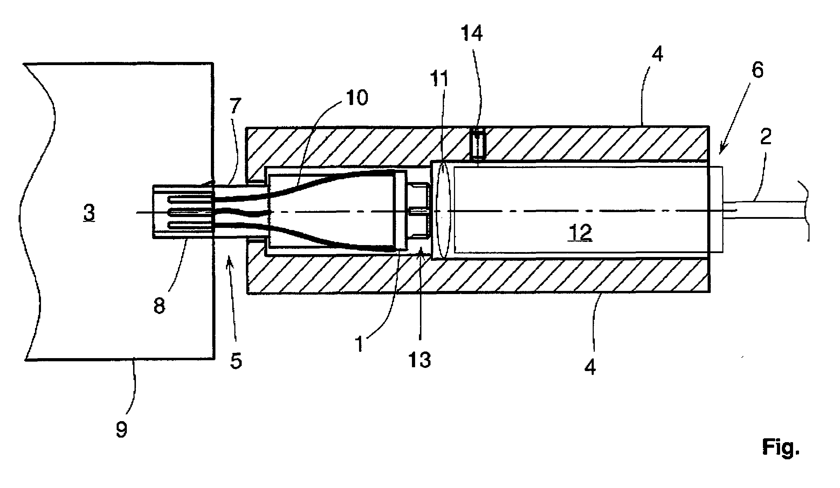

Es gibt nun verschiedene Möglichkeiten, die Lehre der vorliegenden Erfindung in vorteilhafter Weise auszugestalten und weiterzubilden. Dazu ist einerseits auf die dem Patentanspruch 1 nachgeordneten Patentansprüche und andererseits auf die nachfolgende Erläuterung eines Ausführungsbeispiels der Erfindung anhand der Zeichnung zu verweisen. In Verbindung mit der Erläuterung des bevorzugten Ausführungsbeispiels der Erfindung anhand der Zeichnung werden auch im Allgemeinen bevorzugte Ausgestaltungen und Weiterbildungen der Lehre erläutert. In der Zeichnung zeigt die
- einzige Figur: in einer schematischen Ansicht, teilweise geschnitten, ein Ausführungsbeispiel einer erfindungsgemäßen Lichtquelle mit angedeuteter Stromversorgung.

Die einzige Figur zeigt andeutungsweise eine erfindungsgemäße Lichtquelle zum Einkoppeln von Licht in ein in der Figur nicht gezeigtes chirurgisches Handgerät, wobei es sich dabei im Konkreten um ein Handgerät zur Anwendung in der Ophthalmologie handeln kann. Die Lichtquelle umfasst ein Leuchtmittel 1, einen sich vom Leuchtmittel 1 erstreckenden Lichtleiter 2 und eine Stromversorgung 3.

Erfindungsgemäß ist das Leuchtmittel 1 in einem adapterartigen Gehäuse 4 angeordnet, wobei das Gehäuse 4 bzw. der Adapter einen Anschluss 5 für die Stromversorgung und einen Anschluss 6 für den Lichtleiter 2 umfasst.

Die einzige Figur zeigt des Weiteren, dass der Anschluss 5 durch einen in das Gehäuse 4 integrierten Stecker 7 gebildet ist, der aus dem Gehäuse 4 herausragt. Der Stecker 7 kommuniziert mit einer entsprechenden Buchse 8, die in ein Gehäuse 9 der Stromversorgung 3 integriert ist. Bei geeigneter Anordnung und Ausgestaltung der beiden Gehäuse 4, 9 lässt sich die elektrische Verbindung so herstellen, dass das adapterartige Gehäuse 4 zur unmittelbaren Anlage an das die Stromversorgung 3 beinhaltende Gehäuse 9 gelangt. Bei entsprechender Abstimmung in Form und Größe lässt sich durch das elektrische Ankoppeln bei Arretierung eine bauliche Einheit bilden, die bei Bedarf wieder lösbar ist.

Innerhalb des adapterartigen Gehäuses 4 ist ein Leuchtmittel 1 in Form einer LED ausgeführt. Die LED ist durch einen Kühlkörper 10 gehalten, der die Wärme unmittelbar nach außerhalb des Gehäuses 4 oder zur Gehäusewand transportiert.

Dem Leuchtmittel 1 ist eine Optik 11 nachgeordnet, die gemeinsam mit einem eingesteckten Lichtleiter 2 bzw. mit einem geeigneten Koppelelement 12 die Einkopplungsstelle 13 zum Ankoppeln des Lichts bildet. Das Koppelelement 12 lässt sich über geeignete Klemmmittel 14 innerhalb des Gehäuses 4 sichern bzw. arretieren.

Hinsichtlich weiterer Merkmale, die sich den Figuren nicht entnehmen lassen, sei zur Vermeidung von Wiederholungen auf den allgemeinen Teil der Beschreibung verwiesen.

Schließlich sei angemerkt, dass das voranstehend erörterte Ausführungsbeispiel lediglich der beispielhaften Erläuterung der erfindungsgemäßen Lichtquelle dient, diese jedoch nicht auf das Ausführungsbeispiel einschränkt.

### Bezugszeichenliste

- 1: Leuchtmittel
- 2: Lichtleiter
- 3: Stromversorgung
- 4: adapterartiges Gehäuse
- 5: Anschluss für die Stromversorgung
- 6: Anschluss für den Lichtleiter
- 7: Stecker (Strom)
- 8: Buchse (Strom)
- 9: Gehäuse (für die Stromversorgung)
- 10: Kühlkörper
- 11: Optik, Linse
- 12: Koppelelement
- 13: Einkopplungsstelle
- 14: Klemmmittel, Mittel zum Arretieren

## Patentansprüche

1. Lichtquelle zum Einkoppeln von Licht in ein medizinisches Gerät, insbesondere in ein chirurgisches Handgerät, vorzugsweise zur Anwendung in der Ophthalmologie, mit einem Leuchtmittel (1), einem sich vom Leuchtmittel (1) erstreckenden Lichtleiter (2) und einer Stromversorgung (3) für das Leuchtmittel (1), wobei das Leuchtmittel (1) in einem adapterartigen Gehäuse (4) angeordnet ist, das einen Anschluss (5) für die Stromversorgung (3) und einen Anschluss (6) für den Lichtleiter (2) umfasst, wobei der Anschluss (5) für die Stromversorgung (3) als dem Gehäuse (4) zugeordneter Stecker (7) ausgebildet ist und wobei die Stromversorgung (3) in einem separaten Gehäuse (9) untergebracht ist,
**dadurch gekennzeichnet, dass** der Stecker (7) in das Gehäuse (4) integriert ist und endseitig aus dem Gehäuse (4) herausragt und dass das separate Gehäuse (9) eine Buchse (8) zum Einstecken des Steckers (7) des das Leuchtmittel (1) umfassenden Gehäuses (4) aufweist.

2. Lichtquelle nach Anspruch 1, **dadurch gekennzeichnet, dass** das Gehäuse (4) einteilig ausgeführt ist.

3. Lichtquelle nach Anspruch 1 oder 2, **dadurch gekennzeichnet, dass** das Gehäuse (4) in Form einer zylindrischen Hülse ausgeführt ist.

4. Lichtquelle nach einem der Ansprüche 1 bis 3, **dadurch gekennzeichnet, dass** die Anschlüsse (5, 6) für die Stromversorgung (3) und den Lichtleiter (2) auf gegenüberliegenden Seiten des Gehäuses (4) angeordnet sind.

5. Lichtquelle nach einem der Ansprüche 1 bis 4, **dadurch gekennzeichnet, dass** der Anschluss (6) für den Lichtleiter (2) als eine in das Gehäuse (4) integrierte Aufnahme für das Koppelende eines Lichtleiters (2) oder eines Lichtleiterbündels ausgeführt ist, wobei
die Aufnahme im Wesentlichen durch die Innenwandung des Gehäuses (4) definiert sein kann,

6. Lichtquelle nach Anspruch 5, **dadurch gekennzeichnet, dass** die Aufnahme Mittel (14) zum Arretieren des Lichtleiters (2) in seiner angekoppelten Position umfasst.

7. Lichtquelle nach Anspruch 6, **dadurch gekennzeichnet, dass** die Mittel (14) als Klemmmittel ausgeführt sind.

8. Lichtquelle nach einem der Ansprüche 1 bis 7, **dadurch gekennzeichnet, dass** das Leuchtmittel (1) als LED oder Anordnung mit mehreren LEDs ausgeführt ist.

9. Lichtquelle nach einem der Ansprüche 1 bis 8, **dadurch gekennzeichnet, dass** dem Leuchtmittel (1) innerhalb des Gehäuses (4) eine Linse (11) oder ein Linsensystem nachgeordnet ist.

10. Lichtquelle nach einem der Ansprüche 1 bis 9, **dadurch gekennzeichnet, dass** dem Leuchtmittel (1) ein Kühlkörper (10) zugeordnet ist.

11. Lichtquelle nach einem der Ansprüche 1 bis 10, **dadurch gekennzeichnet, dass** die Buchse (8) in das Gehäuse (9) der Stromversorgung (3) integriert ist.

12. Lichtquelle nach einem der Ansprüche 1 bis 11, **dadurch gekennzeichnet, dass** das Gehäuse (4) des Leuchtmittels (1) und das separate Gehäuse (9) der Stromversorgung (3) in etwa aneinander angepasst sind.

13. Lichtquelle nach Anspruch 12, **dadurch gekennzeichnet, dass** die beiden Gehäuse (4, 9) im zusammengesteckten Zustand eine bauliche Einheit ergeben.

14. Lichtquelle nach einem der Ansprüche 1 bis 13, **dadurch gekennzeichnet, dass** die Stromversorgung (3) in deren Gehäuse (9) einen oder mehrere Akkus umfasst.

## Claims

1. Light source for coupling light into a medical device, in particular into a hand-held surgical device, preferably for use in ophthalmology, having a lamp (1), a light guide (2) extending from the lamp (1), and a power supply (3) for the lamp (1), wherein the lamp (1) is arranged in an adapter-like housing (4) which comprises a connection (5) for the power supply (3) and a connection (6) for the light guide (2), wherein the connection (5) for the power supply (3) is in the form of a plug (7) associated with the housing (4), and wherein the power supply (3) is accommodated in a separate housing (9),
**characterised in that** the plug (7) is integrated into the housing (4) and protrudes from an end of the housing (4), and **in that** the separate housing (9) has a socket (8) for insertion of the plug (7) of the housing (4) comprising the lamp (1).

2. Light source according to claim 1, **characterised in that** the housing (4) is in one piece.

3. Light source according to claim 1 or 2, **characterised in that** the housing (4) is in the form of a cylindrical sleeve.

4. Light source according to any one of claims 1 to 3, **characterised in that** the connections (5, 6) for the power supply (3) and the light guide (2) are arranged on opposite sides of the housing (4).

5. Light source according to any one of claims 1 to 4, **characterised in that** the connection (6) for the light guide (2) is in the form of a receiver, integrated into the housing (4), for the coupling end of a light guide (2) or of a bundle of light guides, wherein the receiver can be defined substantially by the inside wall of the housing (4).

6. Light source according to claim 5, **characterised in that** the receiver comprises means (14) for locking the light guide (2) in its coupled position.

7. Light source according to claim 6, **characterised in that** the means (14) are in the form of clamping means.

8. Light source according to any one of claims 1 to 7, **characterised in that** the lamp (1) is in the form of an LED or an arrangement having a plurality of LEDs.

9. Light source according to any one of claims 1 to 8, **characterised in that** a lens (11) or a lens system is arranged downstream of the lamp (1) inside the housing (4).

10. Light source according to any one of claims 1 to 9, **characterised in that** a heat sink (10) is associated with the lamp (1).

11. Light source according to any one of claims 1 to 10, **characterised in that** the socket (8) is integrated into the housing (9) of the power supply (3).

12. Light source according to any one of claims 1 to 11, **characterised in that** the housing (4) of the lamp (1) and the separate housing (9) of the power supply (3) are matched approximately to one another.

13. Light source according to claim 12, **characterised in that** the two housings (4, 9), when fitted together, result in a structural unit.

14. Light source according to any one of claims 1 to 13, **characterised in that** the power supply (3) comprises one or more batteries in the housing (9) thereof.

## Revendications

1. Source de lumière destinée à injecter de la lumière dans un instrument médical, en particulier un instrument chirurgical manuel utilisé de préférence en ophtalmologie, comportant un moyen lumineux (1), un guide d'ondes optiques (2) s'étendant à partir du moyen lumineux (1), et une alimentation électrique (3) pour le moyen lumineux (1), ledit moyen lumineux (1) étant agencé dans un boîtier (4) en forme d'adaptateur muni d'un raccord (5) pour l'alimentation électrique (3) et d'un raccord (6) pour le guide d'ondes optiques (2), ledit raccord (5) pour l'alimentation électrique (3) étant réalisé sous la forme d'une fiche mâle (7) associée au boîtier (4), et ladite alimentation électrique (3) étant logée dans un boîtier (9) séparé,
**caractérisée en ce que** la fiche mâle (7) est intégrée dans le boîtier (4) et s'avance hors dudit boîtier (4) au niveau d'une extrémité, et **en ce que** le boîtier (9) séparé est muni d'une fiche femelle (8) pour l'enfichage de la fiche mâle (7) du boîtier (4) contenant le moyen lumineux (1).

2. Source de lumière selon la revendication 1, **caractérisée en ce que** le boîtier (4) est réalisé d'une seule pièce.

3. Source de lumière selon la revendication 1 ou 2, **caractérisée en ce que** le boîtier (4) est réalisé sous la forme d'une gaine cylindrique.

4. Source de lumière selon l'une quelconque des revendications 1 à 3, **caractérisée en ce que** les raccords (5, 6) pour l'alimentation électrique (3) et le guide d'ondes optiques (2) sont disposés sur des côtés opposés du boîtier (4).

5. Source de lumière selon l'une quelconque des revendications 1 à 4, **caractérisée en ce que** le raccord (6) pour le guide d'ondes optiques (2) est réalisé sous la forme d'un logement, intégré dans le boîtier (4) et destiné à recevoir l'extrémité de couplage d'un guide d'ondes optiques (2) ou d'un faisceau de guides d'ondes optiques, ledit logement pouvant être défini sensiblement par la paroi intérieure du boîtier (4).

6. Source de lumière selon la revendication 5, **caractérisée en ce que** le logement comprend des moyens (14) destinés à immobiliser le guide d'ondes optiques (2) dans sa position couplée.

7. Source de lumière selon la revendication 6, **caractérisée en ce que** les moyens (14) sont réalisés sous forme de moyens de serrage.

8. Source de lumière selon l'une quelconque des revendications 1 à 7, **caractérisée en ce que** le moyen lumineux (1) est réalisé sous forme de diode électroluminescente ou d'un agencement de plusieurs diodes électroluminescentes.

9. Source de lumière selon l'une quelconque des revendications 1 à 8, **caractérisée en ce qu'**une lentille (11) ou un système de lentilles est disposé à l'intérieur du boîtier (4), en aval du moyen lumineux (1).

10. Source de lumière selon l'une quelconque des revendications 1 à 9, **caractérisée en ce qu'**un corps de refroidissement (10) est associé au moyen lumineux (1).

11. Source de lumière selon l'une quelconque des revendications 1 à 10, **caractérisée en ce que** la fiche femelle (8) est intégrée dans le boîtier (9) de l'alimentation électrique (3).

12. Source de lumière selon l'une quelconque des revendications 1 à 11, **caractérisée en ce que** le boîtier (4) du moyen lumineux (1) et le boîtier séparé (9) de l'alimentation électrique (3) sont sensiblement ajustés l'un à l'autre.

13. Source de lumière selon la revendication 12, **caractérisée en ce que** les deux boîtiers (4, 9), à l'état enfiché l'un dans l'autre, forment une unité de construction.

14. Source de lumière selon l'une quelconque des revendications 1 à 13, **caractérisée en ce que** l'alimentation électrique (3) comporte un ou plus accumulateurs dans son boîtier (9).
